# EUROPEAN PATENT APPLICATION

(11) **EP 4 285 934 A1**
(43) Date of publication of application: **06.12.2023**
(21) Application number: 22176225.5
(22) Date of filing: 30.05.2022
(51) Int. Cl.: A61K 47/68, C08B 37/00, C12P 19/28, C12P 21/00

(54) **MODIFIED TRIMANNOSE-OLIGOSACHARIDES, BISECTED N-GLYCANS COMPRISING SAID TRIMANNOSE CORE AND METHOD FOR OBTAINING THEM**

(71) Applicant: CER Groupe, 6900 Aye (BE); Université de Namur, 5000 Namur (BE)
(72) Inventor: CHARTIER, Léa, 6900 AYE (BE); MAREGA, Riccardo, 6900 AYE (BE); VINCENT, Stéphane, 5000 Namur (BE)
(74) Representative: AWA Benelux

(57) **Abstract**

The present invention is related to a reactive compound being made of modified trimmanose-oligosaccharides, to its preparation method and its use for improving the characteristics of this first material or molecule of interest, especially in therapeutic or diagnostic applications. In particular, the present invention is related a reactive compound comprising a trimannnose core of formula 1 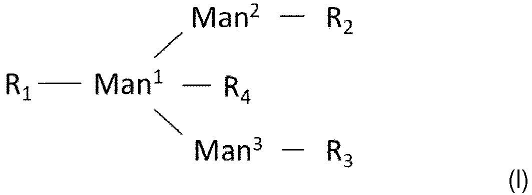 wherein Man¹, Man² and Man³ moieties are a first, a second and a third mannose, respectively, R₁ is hydrogen or a linking group able to bind the first mannose to a ligand being a first material or molecule of interest, R₂ and R₃ are monosaccharides or derivatives thereof, optionally linked to one or more additional saccharide(s), and R₄ is an unnatural monosaccharide group able to bind to a ligand being a second material or molecule of interest different from the first material or molecule of interest. The invention is further related to a method to produce such a reactive compound.

## Description

### Field of the Invention

The present invention is in the field of biotechnology and is related to a reactive compound being made of modified trimmanose-oligosaccharides able to bind to one or more ligand being a material or molecule of interest, to its preparation method and its use for improving the characteristics of this material or molecule of interest, especially in therapeutic or diagnostic applications.

### Background of the invention

Structural modifications of a molecule or material of interest, are mostly based on the reactivity of carboxyl, amino, hydroxyl or thiol groups, which can be contemporaneously and advantageously repeatedly present in the molecule or the material of interest (e.g. protein, saccharide, lipid). This leads to structural heterogeneity in term of number of products (molecules with one or more modifications and addition of ligand(s)) and isomerism (difference in the spatial arrangement of the modification).

Among these examples of materials of interest, (monoclonal or polyclonal) antibodies are known to be functionalized with one or more ligand(s) being molecular probes, drugs or fluorophores, through conjugation protocols involving cysteine or lysine residues, naturally present on the polypeptide framework.

These modifications typically give new properties to these modified antibodies, such as an improved function or an improved reactivity, useful for diagnostic or therapeutic purposes. Efficient and useful modification of a material or a molecule of interest may consist into stochastic modifications. Nevertheless, these modifications can lead to reduce binding/functional properties compared to the unmodified precursor, because a significant fraction of the different structures generated by a procedure that will bear a modification (ligand addition) close to a functional site (e.g. the antigen binding fragment of an antibody).

However, among treated materials or molecules of interest, known stochastic protocols typically lead to antibody batches that are heterogeneous in terms of number and spatial distribution of the payloads. Moreover, the resulting antibodies are known to have lower activity, when compared to their unmodified counterparts, because this stochastic functionalization can occur on the variable regions of these antibodies which include functional sites responsible for the antigen recognition.

On the other hand, site-specific modifications may result into higher structural homogeneity, greater biological activity and easier further functionalization than modifications originating from the stochastic ones. Consequently, the obtained modified material or molecule of interest become therefore more suitable candidates for both diagnostic and therapeutic purposes.

Site-specific modifications can be induced either in-vivo/in-vitro (e.g. by genetic manipulation at the whole organism or cellular levels prior to the bio-production of the molecule) or ex-vivo/ex-vitro (e.g. chemical manipulation of the purified molecule). While the in-vivo/in-vitro strategies are ideal solutions for specific cases and individual products, the ex-vivo/ex-vitro ones are much more broadly applicable (case-independent) and less demanding in terms of timing and economic resources.

Among modified materials of interest, therapeutic proteins such as antibodies and the like are now widely used in diagnostic or therapeutic applications. In particular antibody-drug conjugates may benefit largely from improvements in terms of efficiency, production simplification, and/or reduced side effects.

There is thus a need to provide new simplified methods and new reactive compounds that can be proposed to modify biological structures of a material or molecule of interest, in order to impart novel properties to the material, such as functions or reactivity, especially for diagnostic or therapeutic applications

### State of the art

It is known that a N-Glycosylation site is present in the heavy chain at the asparagine at position 297 (Asn297) of naturally occurring antibodies and two di-antenna shaped glycan moieties made of seven saccharides are formed at this glycosylation site of the antibodies.

The publication of Van Geel, R. et al. (Bioconjug. Chem. 26, 2233-2242 (2015)), .as well as Cai, X. & Janda, K. D. A (Tetrahedron Lett. 56, 3172-3175 (2015)) describe chemoenzymatic approaches to prepare antibody-drug conjugates through the targeting of native glycosylation sites present within antibodies, requiring an enzymatic labelling of native glycans with azide bearing N-Azidoacetylgalactosamie (GalNAz) by addition of endo-beta-N-acetylglucosaminidase.

Terminal galactoses are known to increase the cytotoxic response due to the complement system. Antibody glycosylation and its impact on the pharmacokinetics and pharmacodynamics of monoclonal antibodies and Fc-fusion proteins are known and bisecting GlcNAc are able to increase the affinity toward FcγRIIIa, thus the antibody dependent cell cytotoxicity (ADCC). (Claudia Ferrara, et al. Biotechnol. Bioeng. 93, 851-861 (2006).

In the case of an antibody, it is known that the degree of labelling (DOL) plays an important role; indeed, an antibody with a too important DOL will be more quickly eliminated and more likely to aggregate as it is more hydrophobic. (Publications of Agarwal, P. & Bertozzi, C. R. Bioconjug. Chem. 26, 176-192 (2015). & Van Geel, R. et al. (2015)).

Engineering of modified branching sites upon antibodies has been proposed in the state of the art to create new functional diversity of the modified antibodies. N-glycosylation sites upon antibodies are located on the asparagine 297 (Asn 297 residue), quite far from the variable area of the antibodies, thus perturbations of the antibody activity due to its paratope are unlikely.

There are only two N-glycosylation sites, which means that the number of payload that can added to glycans can be efficiently controlled, which allows a better ratio regulation and homogeneity.

US 11,085,062-B2 patent, European patent EP2305314-B1 (Ratiopharm), European patent EP 1987068-B1 (Life technologies), International patent application WO2014/065661 (Synaffix) and International patent application WO2016/170186 (Genovis) disclose methods for obtaining a conjugate between antibodies and modifying groups.

However, in these patent applications, modifying groups are not added at the bisection position of the trimannose core (bisecting mannose or Man¹), because glycan modification by Glyclick^{®} of Genovis is trimming to the first GlcNac, with addition of GalNAz, with a degree of labelling (DOL) of 2, glycan modification of SiteClick ^{®} of Thermofisher describes a glycan trimming to G0 through addition of GalNAz, with a degree of labelling (DOL) of 4, and glycan modification of GlycoConnect ^{®} (Synaffix) describes a glycan trimming to the first GlcNac, with addition of GalNAz, with a degree of labelling (DOL) of 2.

### Aims of the Invention

The present invention aims to provide new reactive compounds that can be used for the modification of a material or a molecule of interest, especially a (first) biological molecule of interest, wherein the new reactive compounds can efficiently form a conjugate between this (first) material or molecule of interest and the reactive compound, and optionally at least one additional ligand being a (second) material or molecule of interest.

A preferred aim of the invention is to obtain a reactive compound that can be efficiently used in the production of new conjugates, wherein the reactive compound is a linkage structure improving the binding of two, preferably two different, materials or molecules of interest, thereby forming this conjugate.

A specific aim of the invention is to obtain such reactive compound, and the conjugate obtained therefrom, to improve the characteristics of a first material or molecule of interest, while overcoming one or more of the drawbacks of the state of the art.

More preferably, the aim of the invention is to obtain, such binding between an element of a solid support and a suitable ligand preferably a biomolecule, which can be used for the cells culture, especially animal cells, including human cells culture, upon this solid support, or for the screening and the recovering of one or more new materials or molecules of interest.

In particular, a preferred aim of the invention is to obtain reactive compounds for obtaining new conjugates comprising addition of one or more ligand(s), such as drugs and characterized by a higher drug loading upon this first material or molecule of interest, such as antibodies and the like, but without drawbacks of conjugates of the state of the art, in particular in terms of homogeneity and reproducibility.

A more preferred aim of the invention is to obtain a conjugate comprising a first material of interest, being an antibody or the like, and characterized by a low degree of labelling (DOL), closed to 2, when modified by the addition of a reactive compound of the present disclosure, and of the added ligand being a second material of interest.

Another aim of the invention is to obtain an efficient production method of the reactive compound, especially a simplified method, comprising less process steps, being less complex or less time consuming than the methods of the state of the art used for obtaining similar conjugates

A further aim of the invention is to obtain improved conjugates, comprising or made of materials or molecules of interest, such as a glycoproteins, preferably antibodies or the like, and linked by a reactive compound to one or more drug(s) or label(s), especially conjugates improved by the addition of ligands fixed by the reactive compound of the invention and presenting improved characteristics when applied in the diagnostic field, but also in the prevention and/or treatment of plant, animal and human diseases, especially cancer.

### Summary of the Invention

A first aspect of the present invention concerns a reactive compound comprising a trimannnose core of formula (1), 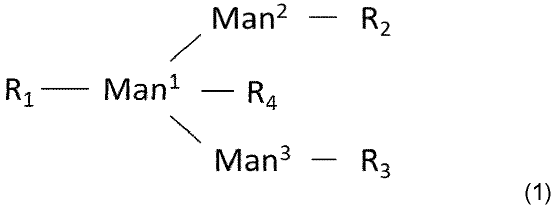 wherein
- Man¹, Man² and Man³ are a first mannose, a second mannose and a third mannose moieties, respectively,
- R₁ is hydrogen or a linking group able to bind the first mannose (Man¹) to a ligand being a first material or molecule of interest,
- R₂ is a monosaccharide or a derivative thereof,
- R₃ is a monosaccharide or a derivative thereof, and
- R₄, is an unnatural monosaccharide group which is able to bind to a ligand, being a second material or molecule of interest.

According to the invention, Man¹ is a bisecting mannose moiety, being at the bisection position of the trimannose core. R₄ is present at the bisection position of the trimannose core and the terminal unnatural monosaccharide in R₄ is preferably a N-Azidoacetylglucosamine (GlcNAz) terminal group

Advantageously, in the reactive compound according to the invention, both ligands (the first material or molecule of interest and the second material or molecule of interest) are different.

According to the invention, R₁ is a linking group (linking the reactive compound to a material or molecule of interest) and this linking group is selected from the group consisting of monosaccharides or derivatives thereof, and disaccharides or derivatives thereof and optionally this linking group further comprises linked to these monosaccharides one or more additional (substituted or not and /or ramified or not) polyethylene glycol (PEG), such as a (OC₂H₄)n chain, wherein n is an integer, preferably comprised between 1 and 20, more preferably between 1 and 10 or between 1 and 5, one more additional alkyl groups, one or more additional aryl groups (from 5 to 12 carbons comprising one or more heteroatoms or not), one or more additional akenyl groups, one or more additional alkene groups, one or more additional alkynes groups or one or more additional alkynyl groups.

Advantageously, R₁ is N-acetylglucosamine (GlcNAc) group or a N-acetylglucosamide - N-acetylglucosamide group (GlcNAc-GlcNAc group). This GlcNAc-GlcNAc group can be further glycosylated, for example is further fucosylated.

Preferably in the reactive compound of the invention, R₁ is a monosaccharide able to bind to the first material or molecule of interest

Optionally, R₂ and/or R₃ is possibly linked to one or more additional saccharide(s), forming saccharide chains..

According to a preferred embodiment of the invention, R₂ and/or R₃ comprises linked to this terminal group, one or more additional (substituted or not and /or ramified or not) polyethylene glycol (PEG), such as a (OC₂H₄)n chain, wherein n is an integer, preferably an integer comprised between 1 and 20, more preferably an integer comprised between 1 and 10 or an integer comprised between 1 and 5, one or more alkyl groups, one more additional aryl groups , aryl groups from 5 to 12 carbons comprising one or more heteroatoms or not, one or more additional akenyl groups, one or more additional alkene groups, one or more additional alkynes groups, or one or more additional alkynyl groups.

Preferably the terminal group of R₂ and/or R₃ being the N-Azido acetylglucosamine (GlcNAz) group(s) able to bind further ligand(s) being a third and optionally a fourth material or molecule of interest.

Furthermore in the reactive compound according to the invention, R₁ can be a disaccharide able to bind linked to the ligand, being the first material or molecule of interest, preferably R₁ is a GlcNAc-GlcNAc group, optionally further fucosylated.

Another aspect of the invention is related to a conjugate formed between at least a first material or molecule of interest and a second material or molecule of interest, and optionally of a third and fourth material or molecule of interest, wherein the materials of interest are bound by the reactive compound according to the invention Advantageously, in this conjugate, the ligands (the at least first material of interest and second material of interest and possibly the third material of interest and fourth material of interest) are different.

In the conjugate according to the invention, the ligand, preferably the material or molecule of interest is a biological molecule selected from the group consisting of a protein, a peptide, a nucleic acid molecule (DNA or RNA sequences such as probes), a saccharide, such as a monosaccharide or a polysaccharide, a lipid or a mixture thereof.

Advantageously, the ligand being the material or molecule of interest in the conjugate according to the invention is a protein is selected from the group consisting of antigenic structure or antibodies and the like, including also monoclonal antibodies, polyclonal antibodies, antibodies hypervariable portions, nanobodies, alphabodies, microbodies, affytins, fymomers, affilines, affimers, or a mixture of two or more thereof.

Advantageously, the ligand being the second material or molecule of interest is selected from the group consisting of a drug, a label, a solid support (e.g. made of glass, polymer or metallic structure), a macromolecule, a carrier molecule, or a water soluble polymer.

The water soluble polymer can be the polyethylene Glycol (PEG), preferably a PEG, having a degree of polymerisation comprised between 1 and about 20.000 and/or having a molecular weight comprised between 1 KDa and 100 KDa, more preferably between 2 KDa and 50 KDa, even more preferably between 5 KDa and 25 KDa, such as a PEG having between 10 KDa and 20 KDa.

It is known that such water soluble polymer, like PEG when added to a biological molecule, such as an antibody and the like can be used to reduce immunogenicity and/or to prolong the clearance time for circulation of the first modified material or molecule (such as this antibody) of interest.

The invention is also related to a pharmaceutical composition comprising a suitable or an adequate pharmaceutical carrier or diluent and the reactive compound and /or the conjugate according to the invention.

Another aspect of the invention concerns a diagnostic or screening kit comprising a reactive compound, and/or the conjugate according to the invention, and optionally comprising means for generating a signal from a label.

The present invention concerns also a solid support, preferably a solid support made of the conjugate of the invention for the screening and the recovery of molecules of interest through binding between molecules of interest and the solid support recovered and fixed to the reactive compound according to the invention. The invention is also related to a support made of the conjugate of the invention for cells culture, especially animal cells, including human cells .

Advantageously, the ligand being the first material of interest in the conjugate according to the invention is provided in the shape of a bead, a strip, a plate or a mixture thereof.

A further aspect of the invention method for producing the reactive compound according to the invention. Advantageously, the method is a chemo-enzymatic method, comprises the steps of:
- selecting a trimannose core of formula 1, or a first material or molecule of interest (such as an antibody) comprising one or more of this trimannose core,
- adding to the trimannose core of formula 1, a sufficient amount of uridine diphosphate N-azidoacetylglucosamine (UDP-GlcNAz), as a donor substrate, and a sufficient amount of a MGAT3 glycosyltransferase (also called Gnt-III or the Beta-1,4-mannosyl-glycoprotein 4-beta-N-acetylglucosaminyltransferase) including a similar enzyme or a variant of this enzyme, presenting a high similarity or nucleotide identity with wild type MGAT3 enzyme, preferably a nucleotide sequence identity higher than 80%, higher than 90%, higher than 95% or higher than 98%-99% with the MGAT3 glycosyltransferase, working as an enzyme to add a terminal N-Azidoacetylglucosamine (GlcNAz) group upon the first mannose Man¹ moiety of the trimannnose core of formula 1, and
- purifying the obtained product, preferably by one or more steps of chromatography, thereby obtaining the reactive compound. or the first material or molecule of interest (antibody) bound to the reactive compound able to bind to a ligand being the second material or molecule of interest

Optionally, in the method of the invention, this first material or molecule of interest is an antibody previously submitted to a modification of one or more of its saccharide chains, for example through the addition of a sufficient amount of a mixture of a Neuraminidase (NAM), a Galactosidase (GO) and an Acetylglucosaminidase (NAG).

A last aspect of the invention is related to a method for producing the conjugate according to the invention. Advantageously, this method comprises a binding of the reactive compound, obtained by the above described method, to a second material or molecule of interest, and possibly to the above described first material or molecule of interest.

Advantageously, in the method according to the invention, the binding is realized by addition of a sufficient amount of the ligand, being this second and first material or molecule of interest to the reactive compound and this method further comprises a subsequent purification step of the obtained conjugate, preferably by one or more step(s) of chromatography

### Brief description of the drawings

The figures 1 and 2 represent structures of preferred examples of the reactive compound according to the invention, whrein M representing the first material or molecule of interest. In the left part of the figures, the legend for the attribution of a monosaccharide structure to a combination of geometric element), according to the Symbol Nomenclature for ligands being glycans (SNFG, Glycobiology 29:620-624, 2019). General structure of the product

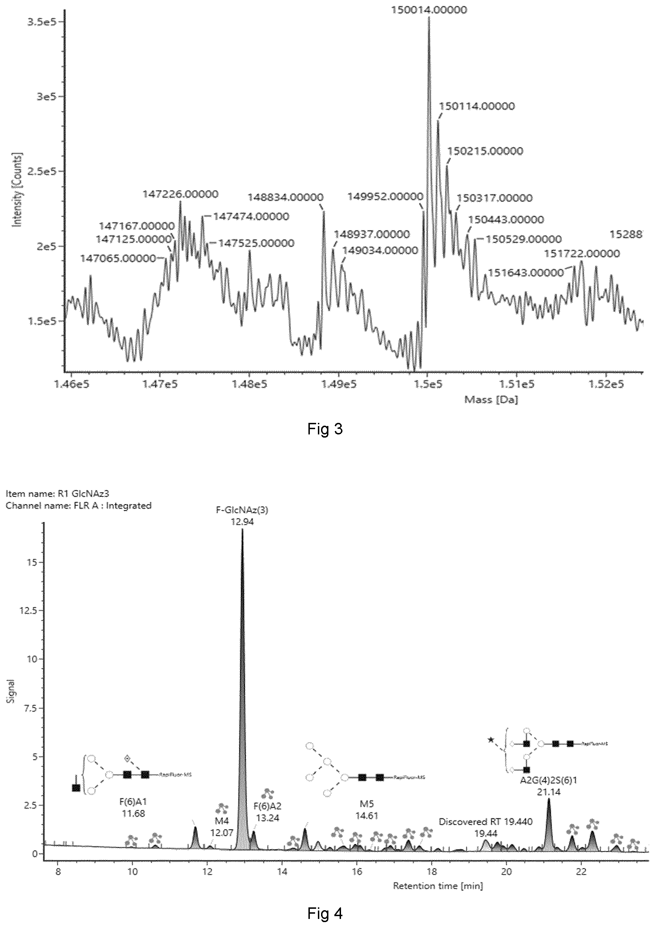

The figure 3 represents an UPLC-FLR chromatogram showing the detection of the reactive compound according to the invention, after treatment by RapiFluor Glycan and analysis by HRMS.

The figure 4 represents MaxEnt1 background subtracted deconvoluted spectrum of F(6)A2BGlcNAz (left) ,showing a main peak with a molecular mass of 150014 Da, corresponding to the reactive conjugate according to the invention.

### Definitions and abbreviations listing

Unless defined otherwise, all technical and scientific terms used herein have the same meanings as commonly understood by one of ordinary skilled in the art to which this invention belongs. All publications and patent documents mentioned herein are incorporated by reference and might be used in connection with the invention.
- ACN: Acetonitrile
- BGG: Bovine Gamma Globulin
- CCD: Charge Coupled Device
- CHO: Chinese Hamster Ovary
- DAR: Drug to Antibody Ratio
- ELISA: Enzyme-Linked Immunosorbent Assay
- ESI: Electrospray lonisation
- FPLC: Fast Protein Liquid Chromatography
- GalT: Galactosyltransferase
- GO: Galactosidase
- HEK: Human Embryonic Kidney
- HILIC: Hydrophilic Interaction Liquid Chromatography
- HPLC: High Pressure Liquid Chromatography
- HRMS: High Resolution Mass Spectrometry
- LC: Liquid Chromatography
- MS: Mass spectrometry
- NAG: N-Acetylglucosaminidase
- NAM: Neuraminidase
- PAGE: PolyAcrylamide Gel Electrophoresis
- PBS: Phosphate Buffered Saline
- SDS: Sodium Dodecyl Sulphate
- SEC: Size Exclusion Chromatography
- SPE: Solid Phase Extraction
- SPI: Soy Protein Isolate
- TLC: Thin Layer Chromatography
- TMB: 3,3',5,5'-Tetramethylbenzidine
- TRIS: Tris(hydroxymethyl)aminomethane
- UDP: Uridine DiPhosphate

The general terms *"saccharide(s)"* or *"glycans"* used herein refer to a natural or unnatural saccharide or glycan including Glucose (Glc), Galactose (Gal), Mannose (Man), Fucose (Fuc), Fructose (Fru), Xylose (Xyl), N-acetylneuraminic acid (Neu5Ac), and N-Glycolylneuraminic acic (Neu5Gc), as well as derivatives thereof, like glucosamine (GlcN), galactosamine (Gain), N-Acetyl-Glucosamine (GlcNAc), N-azidoacetylglucosamine (GlcNAZ), N-Acetylgalactosamine (GlaNAc), (tri)mannose, N-glycan, and azido mannose.

The terms *"trimannose core"* refer to a tri-mannosyl core comprising three connected mannoses (Man¹, Man² and Man³⁾ moieties as represented in formula (1) hereafter, wherein two mannoses (Man² and Man³⁾ moieties are linked to a first mannose (Man¹) moiety being at the bisectional position between the two others mannoses (Man² and Man³ ) moieties through an alpha-1,3 glycosidic linkage or an alpha-1,6 glycosidic linkage.

The term "material (M)" means any solid support or molecule that could be added as a ligand to the reactive compound of the invention and bound as one or more of (or through one or more of ) the moieties R₁, R₂, R₃ and/or R₄,

The terms *"biological molecule"* refer to biological molecules that can be synthetized or purified and that have advantageous targeting properties. Preferably the biological molecules are able to bind other biological molecules, such as proteins and peptides, targeting other similar biological molecules, such as antigens or receptors. Alternatively, and also preferably, these *"biological molecules"* are selected from the group consisting of saccharides, including polysaccharides, nucleic acids, such as DNA or RNA sequences or specific nucleic acid probes of several hundred nucleotides able to bind complementary sequences, lipids or a mixture of two or more thereof.

The term *"protein"* is including polypeptides or peptides molecules with an amino-acid sequence, having a native amino acid sequence, as well as variants and modified forms, regardless of their origin or mode of preparation. These proteins are possibly modified by addition of groups to form glycosylated proteins, by addition of N-linked, O-linked and/or C-linked glycans and/or phosphorylated proteins. Preferably, these proteins are therapeutic moieties and/or targeting moieties, such as antibodies and the like, cytokines, growth factors, hormones, interferons, blood coagulation proteins, Erythropoietin (EPO), G-coupled receptors (GPCRs) or molecules able to bind these receptors, enzymes, such as glucosaminyltransferase, galactosidase, neuraminidase, acetylglucosaminidase, mannosidases, mannosyltransferase, Urokinase, and DNAse.

The terms *"antibody and the like"* are proteins including monoclonal chimeric antibodies or polyclonal antibodies (in particular obtained from serum of from homo- or hetero-hybridoma), and immunoglobulins or specific fragments of these antibodies, such as the hypervariable portion(s) of an antibody or the so-called Immunoglobulins Variable Domains (IVDs), including the Complementary Determining Regions (CDR) of an antibody, preferably Fab fragments, such as F(ab) fragment, F(ab')2 fragment, Fv fragment, Fc fragment and scFv-Fc fragment, but also similar targeting molecules presenting the same specificity and targeting properties of an antibody. Preferably, the targeting antibody and the like protein is selected from the group consisting of diabodies, minibodies, humanized antibodies, pegolyzated antibodies, nanobodies, alphabodies, microbodies, affytins, fymomers, affilines, affimers, or a mixture of two or more thereof.

Antibodies are proteins generated by the immune system and are capable of recognizing and binding to a specific antigen. Due to their properties of sensitivity and specificity, they are often used as molecular tools for research and diagnostic purpose, but also for targeting specific sites (i.e. epitopes) in therapy or prophylaxis.

Antibodies are preferred examples of the first ligand being a biological material of interest to form the conjugate of the invention, because they often present the trimannose core of the reactive compound of the invention. This configuration allows the skilled person to incorporate directly an N-Azidoacetylglucosamine group at position R⁴, upon the first mannose (Man¹) moiety as above described.

Preferred examples of antibodies are the ones that may target tumour cells and can be used against cancer. These molecules can be produced by a different type of prokaryotic or eukaryotic cells, preferably CHO or HEK293 cells, and can be modified to express a type of antibody (i.e. immunoglobulin type) targeting a specific antigen or a specific epitope of an antigen.

Preferred examples of proteins are tumour necrosis factor alpha receptor/igG fusion peptides, chimeric anti-glycoprotein IIb/IIIa antibodies, chimeric anti-Her2 antibodies, chimeric anti-respiratory syncytial virus antibody, chimeric anti-CD20 antibodies, chimeric anti-tumour necrosis factor antibodies, anti-RSV antibodies, anti-I L2 antibodies, and anti-CEA antibodies.

Preferred examples of antibodies are Cetuximab ^{®} (CTX) (commercialized as Erbitux ^{®}) targeting epidermal growth factor receptor (EGFR) and used to treat or to prevent colorectal, head and neck cancers, and Trastuzumab ^{®} (commercialized as Herceptin ^{®}) and targeting HER2 receptor, used in the treatment or prevention of specific type of breast cancers.

Others examples of proteins or molecules that could be modified by the addition of the reactive compound of the invention are glucosaminyltransferase, GnT 1, GnT2, GnT3, MGAT3, galactosidase, neuraminidase, acetylglucosaminidase, mannosidase, mannosyltransferase, Alg 2, UDP-GlcNAz, GDP-ManAz, ....

The terms *"solid support"* refer to any support made of any solid material, but preferably a material selected from the group consisting of elements of a binding pair, metallic support, polysaccharide support (such as gels), glass support, a ceramic material or polymeric support, such as homopolymers and copolymers, a composite, or combinations of two or more thereof. This solid support can have any shape or format, such as beads, including magnetic beads, strips, plates or a mixture thereof. The solid support is preferably made of a material selected from the group consisting of glass, a polymeric structure, such a latex particles or beads, a metal or alloy of metals, such as metallic particles or metallic beads, including magnetic particles or magnetic beads, silicon particles or silicon beads.

This solid support can be modified to include linking groups, one or more additional (substituted or not and /or ramified or not) polyethylene glycol (PEG), such as a (OC₂H₄)n chain, wherein n is an integer, preferably comprised between 1 and 20, more preferably between 1 and 10 or between 1 and 5, one or more additional alkyl group(s) one more additional aryl group(s) (from 5 to 12 carbons comprising one or more heteroatoms or not) one or more additional akenyl group(s), one or more additional alkene group(s), one or more additional alkynes group(s), or one or more additional alkynyl group(s) to bind to a material of interest, especially biological molecules of interest, such as proteins.

The terms *"reactive compound"* mean a chemical compound made of saccharides (here in particular a tri-mannose core made of Man¹, Man² and Man³ presented in formula 1, and being able to bind to a first material of interest or a solid support, to form a conjugate and to improve the characteristics of this first material of interest or to create new binding possibilities of this first material of interest or to the target molecule it could bind, especially to allow the created conjugate to bind to one or more other target molecules, such as new antigenic structures.

The terms *"conjugate"* and *"gluco-conjugate"* refer to a first material or molecule of interest, such as a protein or an element of a solid support, that is linked to a reactive compound, advantageously by a covalent binding, and optionally to at least one other second material or molecule of interest, which is advantageously different from the first material or molecule of interest.

The term "label" refers to a chemical or biochemical group that can be easily detected through its molecular size and/or that is able to generate a detectable signal, such as a fluorescent label , preferably a fluorophore including, but not limited to, cyanine-like and fluorescein-like, a chromophoric label, an electron dense label, a chemo luminescent label, a radioactive label, an enzymatic label, being preferably an enzyme including, but not limited to, HRP, AP and β-lactamase, a positron emitter, a metallic particle preferably colloidal gold, able to react with silver particles to create a colorimetric detectable label, or a first member of a binding pair.

Preferably, the binding pair is made of commercially or synthetically accessible derivatives of binding pairs, preferably made of biotin molecule or iminobiotin molecule, being the first member of the binding pair and able to bind specifically to the second member of the binding pair, being avidin or streptavidin, or antibody-hapten couples (including, but not limited to digoxigenin, dinitrophenol, FAM...).

The term *"drug"* means in the light of the present invention, a therapeutic molecule able to cure or to kill a target cell, preferably a target human cell, such as a tumour cell. These drugs are cytostatic or cytotoxic agents well known by the skilled person. Preferably, this drug is selected from the group consisting of Maytansinoids (derived from macrolide Maytasine from *Maytenus* plants), auristatins (derived from Dolastatin peptides), calicheamicins (enediyene antibiotics from *Micromonospora echinospora* bacteria), antimetabolites, such as, without being limited thereto, florouracil, floxuridine, methotrexate, leucovorin, hydroxyurea, thioguanine, mecaptopurine, cytarabine, pentostatin, fludarabine phosphate, cladribine, asparaginase, gemcitabine, capecitibine, azathioprine, cytosine methotrexate, trimethoprim, pyrimethamine, or permetrexed, alkylating agents, such as, without being limited thereto, emelphalan, chlorambucil, busulfan, thiotepa, ifosfamide, carmustine, lomustine, semustine, streptozocin, dacarbazine, mitomycin C, cyclophosphamide, mechlorethamine, uramustine, dibromomanitol, tetranitrate, procarbazine, altretamine, mitozolomide, or temozolomide, anthracyclines, such as, without being limited thereto, daunorubicin, doxorubicin, eprubicin, idarubicin, orvalrubicin antibiotics, such as, without being limited thereto, dactnomycin, bleomycin, mithramycin, anthramycin, streptomycin, or gramicidin D, calicheamicins, antimitotic agents, dolostatins, cryptophycins, vinca alkaloids, such as, without being limited thereto, vincristine, vimblastine, vindesine, or virorelbine, taxanes, such as, without being limited thereto, paclitaxel or docotaxel topoisomerase inhibitors, such as, without being limited thereto, irinotecan, topotecan, camptothecin, etoposide, teniposide, amsacrine, or mitoxantrone, proteasomes inhibitors, such as, without being limited thereto, peptidyl boronic acid, HDAC inhibitors, such as, without being limited thereto, vorinostat, romidepsin, chidamide, panobinostat, or belinostat, radioisotopes, radioactive agents, salts of these molecules or a mixture of two or more thereof.

According to the invention, the drug is any suitable cytototoxic compound, such as Auristatin E, taxol, cytochalasin B, Gramicidin D, ethidium bromide, emetine, mitomycin, etoposide, tenoposide, vincristine, vinblatine, colchicine, doxorubicin, daunorubicin, dihydroxy anthracinedione, mitoxantrone, mithramycin, actinomycin D, procaine, tetracaine, lidocaine, propranolol, puromycin, immunotoxins, such as endotoxin A of pseudomonasor, and salts, analogs or homologs of these molecules.

Conjugates between such drugs and antibodies are called *"antibody-drugs conjugates"* or *ADCs.* ADCs are typically characterized by an increase therapeutic effect and/or a lower toxicity than the natural drug. Examples of ADCs known in the art, include gemtuzumab-ozogamicin ^{®} used in leukaemia cancer therapy, inotuzumab ozogamacin ^{®} used against lymphoma, Trastuzumab emtansine ^{®} used against breast cancer, Kadcyla ^{®} derived from Trastuzumab ^{®} used to treat breast cancer, mylotarg ^{®}, besposa ^{®}, and adcetris ^{®}.

In addition, the drug in the light of the present invention can be selected from the group consisting of antimicrobial molecules (i.e. especially against parasites, such as trypanosomae species, or plasmodium species), antibacterial molecules or antiviral molecules.

The terms *"radioactive agent"* include any effective radioactive molecule used in diagnosing and/or for destroying a cell, tissue or organ affected by a disease, in particular a portion of a tumour or a tumour or a tumour cell or tissue in its entirety.

Preferably, the radioactive agent is selected from the group consisting of Indium-111, Yttrium-90, Phosphorus-32, Bismuth-212, lodine-131, lodine-123, Cobalt-60, Technetium, Luteniu-177 or a mixture thereof.

The term *"disease"* refers to a state of health of an animal, wherein the health of this animal, including a human, is decreasing. Preferably, this disease is generated by cells having hyper-proliferative characteristics and/or deleterious effects upon animal and human health, such a tumour cells and tissues.

The terms *"chemo-enzymatic method"* refer to a production method of a reactive compound or a conjugates according to the invention, wherein the production method involves one or more enzymatic reactions. Such enzymatic reactions are usually specific in terms of chemo-activity, targeted region and/or stereo specificity, which ensures structural homogeneity of the obtained products.

The term "enzyme MAGT3 "refers to any enzyme encompassed by the invention that has less than 100% sequence identity or sequence identity with specifically wild type MAGT3 enzyme sequence , but presenting the same activity: ability to add a terminal N-Azidoacetylglucosamine (GlcNAz) group upon the first mannose Man¹ of the trimannnose core of formula 1.

"Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988)

Preferred methods to determine identity are designed to give the largest match between two or more sequences tested. Methods to determine identity and similarity are codified in publicly available computer programs. Preferred computer program methods to determine identity and similarity between two sequences include e.g. the GCG program package (Devereux, J., et al, Nucleic Acids Research 12 (1): 387 (1984)), BestFit, BLASTP, BLASTN, and FASTA (Altschul, S. F. et al, J. Mol. Biol. 215:403- 410 (1990). The BLAST X program is publicly available from NCBI and other sources (BLAST Manual, Altschul, S., et al, NCBI NLM NIH Bethesda, MD 20894; Altschul, S., et al, J. Mol. Biol. 215:403-410 (1990). The well-known Smith Waterman algorithm may also be used to determine identity;

The present invention will be further described in the following detailed description of the invention and examples in reference to the enclosed figures both being presented as non-limiting preferred embodiments of the invention.

### Detailed Description of the Invention

The present invention concerns a new reactive compound made of an oligosaccharide structure based on a trimannose core (also named tri-mannosyl core) bearing one or more new "clickable" units according to the general structure of formula 1 as described above.

This new configuration comprises at the bisection position of the trimannose core being the bisecting mannose, or Man¹ moiety in formula 1, an unnatural monosaccharide R₄. Advantageously, R₄ preferably is N-Azidoacetylglucosamine (GlcNAz) which is able to bind to a suitable ligand, preferably a material or molecule of interest.

The inventors have discovered that such a new configuration is very advantageous, because it allows to generate new "clickable" units and allows to obtain the binding of a ligand being a material being a solid support or a molecule of interest at this position.

As represented in formula 1 and in the figures, the obtained reactive compound is similar to a schematic scorpion, wherein the scorpion mouth is advantageously the bisecting mannose (or Man¹) moiety modified as above described.

Advantageously, the two other mannoses Man² and Man³ moieties can be modified similarly, for example to add further ligands, being materials or molecules of interest. These further ligands being materials or molecules of interest can be different or the same, and can be as the same as the above mentioned added ligands being the material or molecule of interest present in the mouth scorpion.

According to the invention, a first material (M) or molecule of interest is advantageously an antibody present upon the extremity of a scorpion tail, for example bound to the trimannose core of formula 1 by R₁. further added ligands being the second material or molecule of interest present in the mouth of the scorpion, the third materoal or molecule of interest and the fourth material or molecule of interest being optionally present at the extremity of one or both of the claw(s) of this schematic scorpion

The ligand (being a material or a molecule of interest) addition to the reactive compound of the invention is advantageously obtained by the so-called "click chemistry".

Unlike classic stochastic coupling protocols, which use organic solvents and toxic reagents (carbodiimide, benzitriazoles, alkenethiols etc...), this invention is advantageously based upon one or more enzymatic reaction(s) occurring in aqueous buffer(s) and in the presence of (bio)catalysts, which are less energy consuming. Even if some reagents have to be produced by organic synthesis, this invention tends to be a more sustainable protocol.

The selected enzyme is advantageously the MGAT3 glycosyltransferase (also called GnT-III or the Beta-1,4-mannosyl-glycoprotein 4-beta-N-acetylglucosaminyltransferase) glycosyltransferase, as enzyme to add a terminal N-Azidoacetylglucosamine (GlcNAz) group upon the first mannose (Man¹ ) moiety of the trimannnose core of formula 1, with optionally cofactors that facilitate this enzymatic reaction (such as a sufficient amount of MnCl₂, TRIS buffer, ...).

### Examples

### Example 1: Production of the conjugate according to the invention

### Step 1: buffer exchange

The antibody (1 eq, Ci 22 µM, 3.3 mg·mL-1, Cf 6.47 µM, 0.97 mg·mL-1, 50 µg) was buffer exchanged into TRIS buffer (100 mM, pH = 7) by dialysis (Mini D-Tube^{™} dialyzers from Merck Millipore, Ref#71504-3) upon 4 buffer changes in a time span of 16 hours.

### Step 2: enzymatic modification.

UDP-GlcNAz (25 eq, Ci 0.1 M, Cf 0.16 mM, 5.4 µg), the enzyme MGAT3 (0.31 eq, Ci 6.9 µM, 0.4 mg·mL-1, Cf 0.8 µM, 0.12 mg·mL-1, 6 µg), and MnCl₂ (204 eq, Ci 0.1 M, Cf 1.3 mM, 8.3 µg), are subsequently added to the buffer exchanged material, and the mixture was incubated overnight at 37°C.

### Step 3: product isolation

To evidence the presence of the product, an ultra-high performance liquid chromatography-high resolution mass spectrometry (UHPLC-HRMS), is used.

The UHPLC-HRMS analysis reported below were done by using an Acquity liquid chromatograph equipped with different columns and coupled to a Xevo G2-XS QTof quadrupole time-of-flight system (Waters) with an Electrospray lonisation (ESI) source.

Proteins containing the product were first diluted to 0.1 mgmL-1 with HPLC grade water, to a final volume of 100 µl before UHPLC-HRMS analysis.

Calibration was performed with Leu-Enkephalin (m/z 555.26). The data were processed using UNIFI ^{®} software (Waters, Milford, MA, USA) and deconvolutions of combined raw spectrum were done with Masslynx ^{®} on MaxEnt1 settings on UNIFI ^{®}. Here 10 µL of sample diluted in DMF/ACN solution was injected into AQUITY UPLC Glycan BEH Amide, 130 Å, 1.7 µm, 2.1x150 mm column (Waters, Ref#186004742) heated at 60°C.

The running conditions used were as follows: mobile phase A is 50 mM ammonium formate solution, pH 4.4 and mobile phase B is 100% Acetonitrile.

Gradient conditions were as follows:
- 0 to 35 min flow rate of 0.4 mL·min-1 25% A, 35 to 36.5 min flow rate of 0.4 mL·min-1 46% A, 36.5 to 43.1 flow rate of 0.2 mL·min-1 100% A, 43.1 to 47.6 min flow rate of 0.2 mL·min-1 25% A and finally 47.6 to 55 min flow rate of 0.4mL·min-1 25% A.

The settings used were as follows: FLR wavelength EX 265/EM 425nm, FLR sampling rate 2 Hz. source temperature= 120 °C; gas flow= 800 L·h-1; capillary voltage= 3kV; cone voltage= 80V; desolvatation temperature= 350°C; positive ion mode; scan mode= positive sensitivity; mass range scanned= 500 to 2,000 m/z. Mass range calibration (Nal, 6.7 µM, 1µg·mL-1) m/z is comprised between 500 and 2,000.

The N-Glycans release and functionalization was realized with the Waters kit GlycoWorks RapiFluor-MS N-Glycan kit ^{®} (Waters, Ref# 176003713), and by adapting the UHPLC-HRMS by coupling to an AQUITY FLR (fluorescence) detector ^{®} (λex=265 nm, λem=425 nm), used to detect the product after its release.

More specifically, the antibody bearing the product (13.3 µM, 2 mg·mL-1, 7.5 µL, 15 µg) was solubilised with the anionic surfactant RapiGest (Waters, Ref# 186001861, 5% w:v in Glycoworks Rapid Buffer, 6 µL) and HRMS grade water (15.3µL).

The solution was mixed by gentle pipetting, and then heated 3 min at 90°C to induce protein denaturation.

The solution was cooled for 3 min, then PNGase F was added (Rapid PNGase F, 1.2 µL) and incubated 5 min at 50°C. The sample was cooled at RT for 3min prior to the addition of the labelling solution (12 µL, 0.16 M, 68.7 mg·mL-1 in DMF).

The mixture was homogenised by pipetting and after 5 min of incubation at RT it was diluted with 358 µL of ACN, for the subsequent separation by the HILIC SPE step.

HILIC SPE step: a GlycoWorks HILIC µelution plate ^{®} was conditioned with HPLC grade water (200 µL per well) then equilibrated with water/ACN (15:85 vol:vol, 200 µL).

The sample was loaded on the wells (400 µL), which were then washed with a solution of formic acid/water/ACN (1:9:90 vol:vol, 2x600 µL) and finally eluted with Glycoworks SPE Elution buffer (200 mM ammonium acetate in 5% acetonitrile, 3x30 µL).

These eluates (90 µL) were further diluted in GlycoWorks ^{®} Sample diluent (DMF/ACN, 30:70, 310 µL). The sample was then treated by HRMS or was first stored at 4°C for at least 3 days or at -80°C for long term storage.

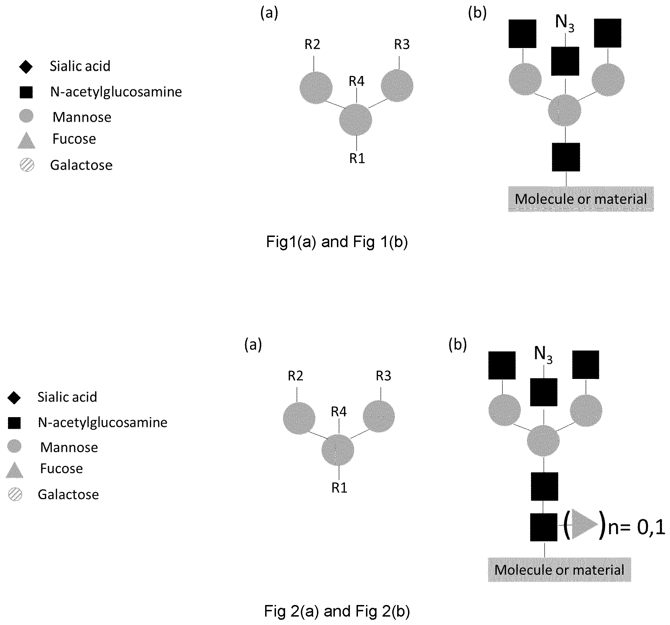

The binding upon an unnatural glucosamine derivative bisecting the trisaccharide core, such as in the modification of the G0(F) glycan (CAS No.84825-26-3) is depicted in Figure 1b, which represents the core form found in glycoproteins and antibodies in most mammalian species and in therapeutic proteins.The binding of one or more of the mannose residues, results into a modification of the N-glycan core pentasaccharide moiety (M3) depicted in the Figures

The reactive compounds according to the invention enable the modulation of biological functions and site selective modifications at two levels of structural hierarchy:
At the oligosaccharide level: derivatives showing bisection of the trimannose core modulate several biological functions: fertility, cancer, neurodegenerative disease, among others), while the presence of unnatural units determines their chemical reactivity (including, but not limited to, bio-conjugations by click-chemistry protocols.
At the macromolecular level: this product can be part of biologically occurring structures, including but not limited to N- and O-glycans, which can be exposed naturally or artificially in spatially defined regions of the molecules or materials of interest, (e.g. a sequence Asn-X-Ser/Thr, in which X can be any amino acid except proline) enabling novel functions such as solubilisation, biological activity and reactivity (site-selective bio-conjugations). This product is intended to industrial use and presents a solution for scientific studies such as:
   - The biological functions of proteins, peptides, glycol-peptides, glycolipids, nucleic acids located inside, onto, or at the exterior of cell membranes (e.g. cell membrane proteins exposing the product);
   - The reactivity of proteins, peptides, glycol-peptides, glycolipids, nucleic acids located inside onto or at the exterior of cell membranes (e.g. cell membrane proteins exposing the product);
   - The necessity of diagnostic and therapeutic products in which the optimal biological activity and the control over the functionalization is key;
   - The biological functions of proteins, peptides, glycol-peptides, glycolipids, nucleic acids that once administered to a biological system (cell, animal, human) act inside, onto, or at the exterior of cell membranes (e.g. therapeutic proteins);
   - The reactivity of proteins, peptides, glycol-peptides, glycolipids, nucleic acids that once administered to a biological system (cell, animal, human) act inside, onto, or at the exterior of cell membranes (e.g. antibody-drug conjugates).

### Example 2: Modulation of biological functions

When the product displays the clickable N-Azidoacetylglucosamine (GlcNAz) as bisecting unit of the trimannose core, it can be used to impart molecules or (nano)materials (from natural or artificial sources) with biological functions as for its natural counterparts lacking of such modification, such as the structures reported in the figures. Such biological functions include, but are not limited to, the modulation of neurodegenerative disease, immune tolerance, IgG functions, tumour metastasis and development, and are detailed below.

### Modulation of neuro-degenerative diseases

Bisecting GlcNAc modifications have shown the capacity to advantageously stabilize BACE1 protein under conditions of oxidative stress (EMBO Mol. Med. 7, 175-189, doi: 10.15252/emmm.201404438). The increased contents of bisecting GlcNAc in AD brains might function as an adaptive response, which protects the brains from the damage caused by additional beta-amyloid yields (Glycobiology 20, 99-106, doi: 10.1093/glycob/cwp152).

The lack of bisecting GlcNAc to BACE1 directed the transport of this protein to the lysosome and accelerated its degradation, which resulted in a lower accumulation of β amyloid in AD (Glycoconj. J. 35, 345-351. doi: 10.1007/s10719-018-9829-4). These findings have highlighted the importance of bisecting GlcNAc modification in the nervous system.

### Modulation of immune tolerance

Bisecting GlcNAc structures have been reported to possess immune suppression functions. For instance, K562 cells are easily killed by natural killer (NK) cells; however, after being transfected with the gene that encodes GlcNAc-T III, K562 cells possessing more bisecting GlcNAc attain NK cell resistance (Mol. Hum. Reprod. 3, 501-505. doi: 10.1093/molehr/3.6.501).

Natural killer (NK) cells are the major type of immune cells found in the human uterus, which indicates that they potentially target sperm (Mol. Hum. Reprod. 20, 185-199. doi: 10.1093/molehr/gat064). Human sperm were found to express bisecting GlcNAc structures, which explains why sperm are not killed by the maternal immune system when entering the female as a foreign substrate and thus support hu-FEDS (Biomed Res. Int. 2019:5397804. doi: 10.1155/2019/5397804).

Additionally, abundant bisecting GlcNAc glycans were detected in human syncytiotrophoblasts (STB) and cytotrophoblasts (CTB) (Mol. Cell. Proteomics 15, 1857-1866. doi: 10.1074/mcp.M115.055798). It is most likely that the maternal immune system was suppressed due to the presence of bisecting GlcNAc glycans and that the foetus benefited from this suppression; the mother could nourish a foetus (similar to a foreign organ as the father contributes to its half genome) within her body for several months without rejection.

The possible mechanism underlying this suppression could be that the glycol-conjugates interacted with lectins that linked to particular signal transduction pathways modulating immune cell functions. For instance, α-2,3-linked sialic acid on soluble CD52, a glycoprotein of 12 amino acids anchored to glycosylphosphatidylinositol, could mediate T-cell suppression by binding to siglec-10 (Front. Immunol. 10:1967. doi: 10.3389/fimmu.2019.0196). It is possible that bisecting GlcNAc can function in a similar way to suppress NK cells.

### Modulation of IgG functions and properties

Fc-glycan has an influence on the biological activities of IgG. For example, a lack of fucose in the Fc glycan significantly improves binding to the human FcyR III, and this result is applied to improve the efficacy of therapeutic monoclonal antibodies. Attachment of bisecting GlcNAc to the Fc glycan will induce antibody-dependent cell mediated cytotoxicity (ADCC).

### Modulation of Tumour metastasis and development

Bisecting GlcNAc structures could inhibit hypoxia-induced epithelial-mesenchymal transition in breast cancer (Front. Physiol. 9:210. doi: 10.3389/fphys.2018.00210). However, the mechanism underlying is still not clear. It has been speculated that the addition of bisecting GlcNAc to the key glycoproteins of signalling transduction, e.g., growth factors, integrins, and cytokine receptors, has its special signalling strength under hypoxia. Actually, observations of nonsolid tumours contradict this explanation. GlcNAc-T III was more activated in patients with chronic myelogeneous leukemia in blast crisis (CML-BC) and in patients with multiple myeloma (MM) (Biochem. J. 331 (Pt 3), 733-742. doi: 10.1042/bj3310733).

When the product displays the clickable mannose, it can be used to impart molecules or (nano)materials (from natural or artificial sources) with biological functions as for its natural counterparts lacking of such modification, such as the structures reported in the figures.

These biological functions include, but are not limited to the modulation of synthetic hormone analogues (Org. Biomol. Chem., 2014,12, 8142-8151, doi: 10.1039/C4OB01208A), binding to HIV and others viruses (J Biol Chem 280: 29269-29276, DOI: 10.1074/jbc.M504642200), glycosylation of therapeutically valuable diabetes, peptide drugs (Front. Chem., 12 2021, doi: 10.3389/fchem.2021.65002), IgG functions, among others.

### Example 3: Modulation of a molecule reactivity

Once the product is part of molecules or (nano)materials from natural or artificial sources, it displays the typical reactivity of azido groups, such as reactions with alkynes and alkenes, phosphines (The Chemistry of the Azido Group ISBN: 978-0-470-77126-6), allowing bio-conjugation with all of the class of molecules of diagnostic and therapeutic interest.

### Reactions with molecules of diagnostic interest

In this respect, commercially or synthetically accessible derivatives of biotin (e.g. including, but not limited to, iminobiotin), fluorophores (e.g. including, but not limited, to cyanine-like, and fluorescein-like), enzymes, including, but not limited to, HRP, AP, and β-lactamase and other antibody-hapten couples including, but not limited to, digoxigenin, dinitrophenol, and FAM, are the references.

### Reactions with molecules of therapeutic interest

In this respect, commercial or synthetical labels, such as accessible derivatives of tracers for in-vivo imaging, including, but not limited to, chelating agents such as DOTA, drugs (including, but not limited to, antitumor compounds, such as monomethyl auristatin E, but also antimicrobial and antiviral molecules, or macromolecules, including, but not limited, to PEG, proteins, lipid, nucleic acids sequences, are the references.

## Claims

1. Reactive compound comprising a trimannose core of formula 1 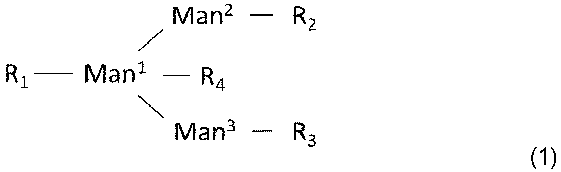 wherein
- Man¹, Man² and Man³ moieties are a first mannose, a second mannose and a third mannose, respectively,
- R₁ is hydrogen or a linking group able to bind the first mannose (Man¹) to a first material of interest,
- R₂ is a monosaccharide, optionally linked to one or more additional saccharide(s),
- R₃ is a monosaccharide, optionally linked to one or more additional saccharide(s), and
- R₄ comprise a terminal unnatural monosaccharide group which is able to bind to a second material of interest different from the said first material of interest.

2. The reactive compound according to claim 1, wherein R₁ is a linking group selected from the group consisting of monosaccharides and disaccharides.

3. The reactive compound according to claim 2, wherein R₁ is N-acetylglucosamine (GlcNAc or GlcNAc-GlcNAc group) group, optionally further fucosylated.

4. The reactive compound according to any one of the preceding claims, wherein the terminal unnatural monosaccharide in R₄, is a N-Azidoacetylglucosamine (GlcNAz) group.

5. The reactive compound according to any one of the preceding claims, wherein R₂ and/or R₃ comprises a terminal a N-Azidoacetylglucosamine (GlcNAz) group

6. A conjugate between at least a first material of interest and a second material of interest, wherein the said first and the said second material of interest are different materials, and wherein the said first and the said second material of interest are bound together by the reactive compound according to any one of the preceding claims.

7. The conjugate according to claim 6, wherein the first material of interest is a biological molecule selected from the group consisting of proteins, nucleic acids, saccharides, lipids or a mixture of two or more there

8. The conjugate according to the claim 7, wherein the protein is selected from the group consisting of monoclonal antibodies, polyclonal antibodies, antibodies hypervariable portions, nanobodies, alphabodies, microbodies, affytins, fymomers, affilines, affimers, or a mixture of two or more thereof.

9. The conjugate according to any one of claims 6 to 8 wherein second material of interest is selected from the group consisting of a macromolecule, a drug or a label.

10. A pharmaceutical composition comprising an adequate pharmaceutical carrier or diluent and a reactive compound according to any one of claims 1 to 5 and/or a conjugate according to any one of claims 6 to 9.

11. A diagnostic kit comprising the reactive compound according to any one of claims 1 to 5 and/or a conjugate according to claim 10, and means for generating a signal from a label.

12. A solid support made of the conjugate according to any one of claims 7 to 9, wherein the first material of interest is a solid support and the second material is a protein

13. The solid support of claim 12, wherein the first material of interest is provided in the shape of a bead, a strip or a plate, for cells culture.

14. A method for producing the reactive compound according to any one of claims 1 to 5, which comprises the steps of
- selecting a trimannose core of formula 1, or a first material of interest comprising this trimannose core;
- adding to the said trimannose core a sufficient amount of uridine diphosphate N-azidoacetylglucosamine (UDP-GlcNAz) as a donor substrate and a sufficient amount of a glycosyltransferase, preferably the MGAT3 glycosyltransferase; as an enzyme to add a terminal N-Azidoacetylglucosamine (GlcNAz) group upon the first mannose Man¹ moiety of the trimannose core of formula 1 and
- purifying the obtained product by chromatography, thereby obtaining the reactive compound.

15. A method for the production of the conjugate according to any of claims 6 to 9, comprising a binding of the reactive compound obtained from the method claim 14 to the second material of interest, and optionally a binding of the reactive compound obtained from method claim 14 to the first material of interest.
